# EUROPEAN PATENT SPECIFICATION

(11) **EP 2 713 907 B1**
(45) Date of publication and mention of the grant of the patent: **01.04.2015**
(21) Application number: 12726972.8
(22) Date of filing: 25.05.2012
(51) Int. Cl.: A61B 17/17

(54) **ADJUSTABLE AIMING ASSEMBLY**
EINSTELLBARE ANVISIERUNGSANORDNUNG
ENSEMBLE DE VISÉE AJUSTABLE

(30) Priority: 25.05.2011 US 201161489930 P
(43) Date of publication of application: 09.04.2014
(73) Proprietor: Synthes GmbH, 4436 Oberdorf (CH)
(72) Inventor: OVERES, Tom, CH-4436 Oberdorf (CH); WALTER, Bruno, CH-4436 Oberdorf (CH)
(74) Representative: Klunker . Schmitt-Nilson . Hirsch
(86) International application number: PCT/US2012/039571
(87) International publication number: WO 2012/162607

(56) References cited:
- EP-A2- 0 589 592
- WO-A1-03/065907
- WO-A2-2006/107222
- US-A- 4 865 025

## Description

### BACKGROUND

To stabilize fractured bones (including such so-called long bones such as femurs, tibias, fibulas, humeri, radii, ulnas, metacarpals, metatarsals, and phalanges), users have employed intramedullary nails to provide structural reinforcement to the bone. Such devices may be anchored, for instance in the medullary canal of the bone, by way of screws inserted through the bone (in a direction transverse to the major axis of the nail) so as to engage with locking holes in the nail.

Some bones, however, have a natural curvature, and intramedullary devices inserted into the medullary canal can deflect so as to conform to this curvature. Accordingly compensation for the curvature of the bone allows for accurate placement of the fixation screws when the screws are aimed and then inserted through the skin and bone to engage with the locking holes of the intramedullary device.

EP 0 589 592 A2 discloses a centering means for holes of intramedullary nails, including a jig having proximal and distal portions. The two portions are pivotally coupled to each other such that holes in the distal portion of the jig can be aligned with holes of an intramedullary nail. An actuating screw is provided in a U-shaped brace on the proximal portion, which engages the distal portion to actuate the hinge. The angle between the proximal and distal portions can be measured by means of a transducer having a spring-loaded pin that contacts the distal portion.

### SUMMARY

The present invention is defined in claim 1. In one embodiment, an aiming assembly includes a base having a spacer member and opposed legs that extend from the spacer member, the opposed legs spaced from each other so as to define a gap between the opposed legs. The aiming assembly also includes an elongate arm that defines a proximal portion and a distal portion that is spaced from the proximal portion along a major axis of the elongate arm. The proximal portion is at least partially disposed in the gap, and the elongate arm defines a plurality of apertures that extend through the distal portion. The aiming assembly further includes a spring that flexibly couples the proximal portion of the elongate arm to the base at a location between the opposed legs, and at least one actuator that is configured to bias the proximal portion of the elongate arm toward one of the opposed legs and away from the other of the opposed legs against a force of the spring.

### BRIEF DESCRIPTION OF THE DRAWINGS

The summary, as well as the following detailed description, is further understood when read in conjunction with the appended drawings. For the purpose of illustrating the present disclosure, there are shown in the drawings example embodiments of the disclosure; however, the disclosure is not limited to the specific methods, compositions, and devices disclosed. In addition, the drawings are not necessarily drawn to scale. In the drawings:
Figs. 1A is a top plan view of an aiming device constructed in accordance with one embodiment;
Fig. 1B is a perspective view of the aiming device illustrated in Fig. 1B;
Fig. 2A is a top plan view of a portion of the aiming device illustrated in Fig. 1A, showing an adjustment assembly;
Fig. 2B is a perspective view of the portion of the aiming device illustrated in Fig. 2A;
Fig. 3A is a top plan view of the aiming device illustrated in Fig. 1A, showing an enlarged adjustment assembly in a first position;
Fig. 3B is a perspective view of the aiming device illustrated in Fig. 3A;
Fig. 4A is a top plan view of the aiming device illustrated in Fig. 1A, showing the adjustment assembly in a second position different than the first position illustrated in Fig. 3A;
Fig. 4B is a perspective view of the aiming device illustrated in Fig. 4A;
Figs. 5A and 5B are an enlarged perspective view and sectional side elevation view of a portion of the aiming device illustrated in Fig. 1A;
Fig. 6A is a perspective view an implantation assembly including a support frame and an intramedullary nail supported by the support frame in accordance with one embodiment;
Fig. 6B is a perspective view of the implantation assembly, showing the intramedullary nail implanted in a long bone;
Fig. 7A is a perspective view of an aiming assembly including the implantation assembly illustrated in Fig. 6A and the aiming arm illustrated in Fig. 1A supported by the support frame;
Fig. 7B is an exploded assembly view of the aiming assembly illustrated in Fig. 7A;
Fig. 8 is a perspective view of the aiming device as illustrated in Fig. 7, further including a mask;
Fig. 9 is a perspective view of the implantation assembly illustrated in Fig. 8, showing the intramedullary nail in an inserted, deflected configuration and showing the aiming arm in a corresponding deflected configuration;
Fig. 10 is a perspective view of the implantation assembly illustrated in Fig. 8, showing the intramedullary nail in another deflected configuration and showing the aiming arm in a corresponding deflected configuration;
Fig. 11 is a perspective view of the implantation assembly as illustrated in Fig. 10, showing the intramedullary nail implanted in a long bone;
Fig. 12 is a top plan view of an aiming device constructed in accordance with another embodiment; and
Fig. 13 is a top plan view of a portion of the aiming device illustrated in Fig. 12, showing an adjustment assembly.

### DETAILED DESCRIPTION

The present disclosure may be understood more readily by reference to the following detailed description taken in connection with the accompanying figures and examples, which form a part of this disclosure. It is to be understood that this disclosure is not limited to the specific devices, methods, applications, conditions or parameters described and/or shown herein, and that the terminology used herein is for the purpose of describing particular embodiments by way of example only and is not intended to be limiting of the scope of the present disclosure. Also, as used in the specification including the appended claims, the singular forms "a," "an," and "the" include the plural, and reference to a particular numerical value includes at least that particular value, unless the context clearly dictates otherwise.

The term "plurality", as used herein, means more than one. When a range of values is expressed, another embodiment includes from the one particular value and/or to the other particular value. Similarly, when values are expressed as approximations, by use of the antecedent "about," it will be understood that the particular value forms another embodiment. All ranges are inclusive and combinable.

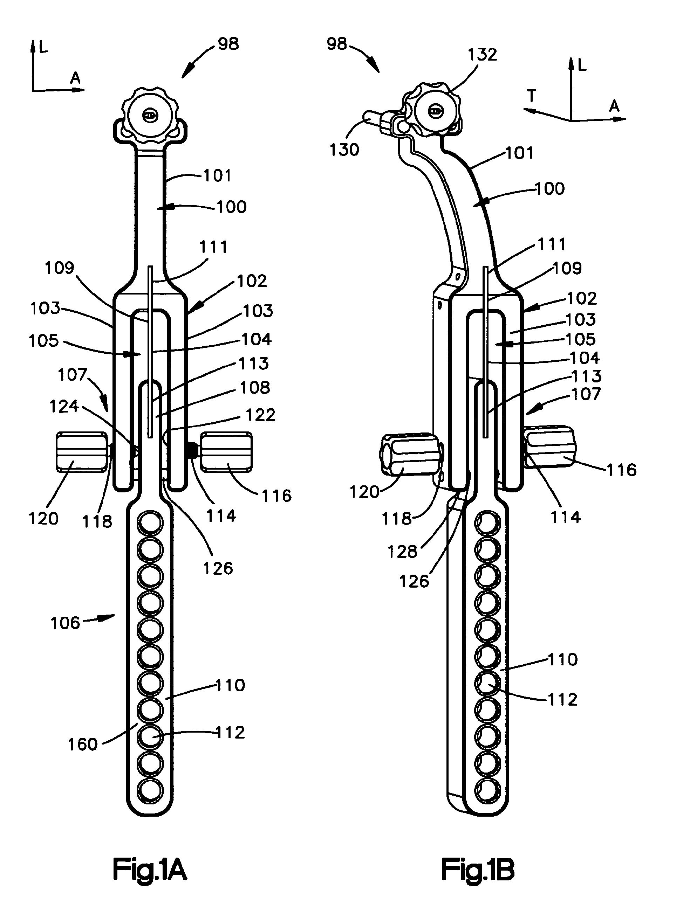

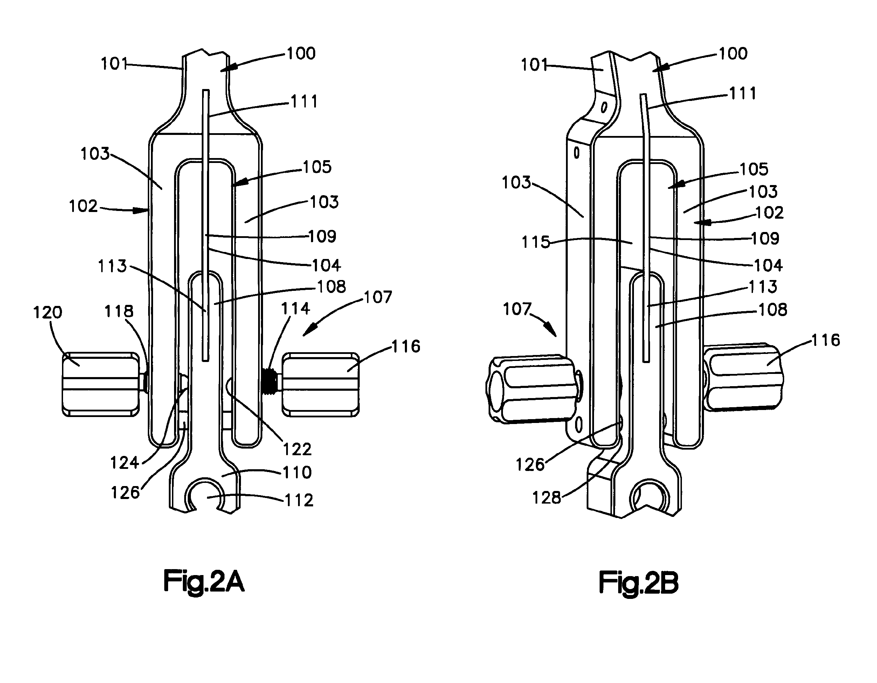

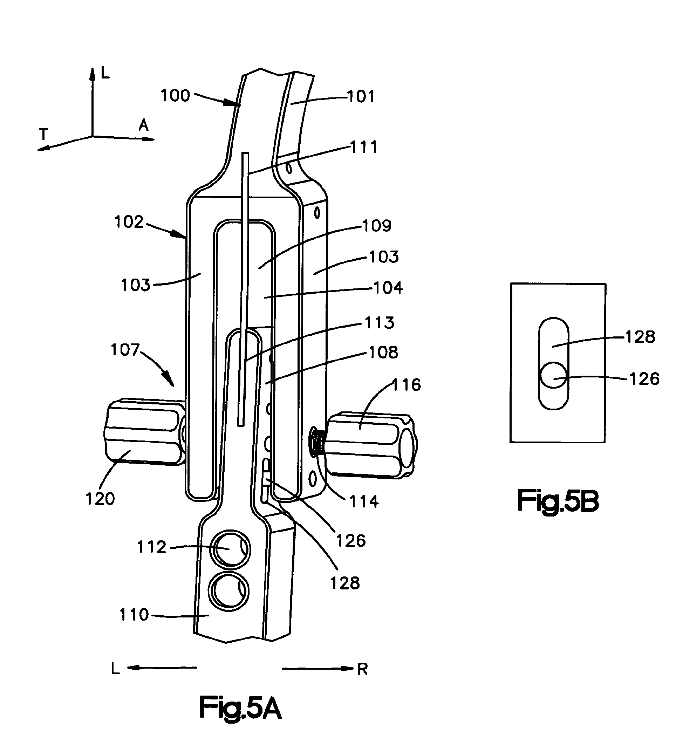

Referring to Figs. 1A-5B generally, an aiming device 98 includes a base 100 and an elongate arm 106 that is supported by the base 100 and movable with respect to the base. The base 100 and the elongate arm 106 are arranged such that the base 100 is disposed proximal with respect to the elongate arm 106. The base 100 of the aiming device 98 is elongate along a central axis 140 that extends along a longitudinal direction L. The base 100 includes a spacer 101 at its proximal end and a forked projection 102 that extends distally from the spacer 101, thus defining the distal end of the base 100. The forked projection 102 includes a pair of opposed legs 103 that are spaced along a lateral direction A that is substantially perpendicular to the longitudinal direction L. Thus, the aiming device 98 includes a length along the longitudinal direction L, a width along the lateral direction A, and a thickness along a transverse direction T that is substantially perpendicular to both the longitudinal direction L and the lateral direction A.

The legs 103 can be spaced and arranged such that the projection 102 defines a C-shape, a U-shape, a Y-shape, or any suitable alternative shape as desired, such that the projection 102 defines a gap 105 that is defined between the legs 103. The elongate arm 106 can be longitudinally elongate having a proximal portion 108 and an opposed distal portion 110 arranged such that the proximal portion 108 is disposed closer to the base 100 than the distal portion 110. The distal portion 110 is spaced from the proximal portion 108 along a major axis of the elongate arm 106. The distal portion 110 can define a lateral width that can be different than, for instance greater than as shown in Fig. 1A or less than, that of the proximal portion 108. Alternatively, the lateral width of the distal portion 110 can be the same as that of the proximal portion 108.

The lateral width of a portion of the elongate arm 106, such as the proximal portion 108, can be less than the lateral width of the gap 105, such that the proximal portion 108 can be positioned in the gap 105 between the legs 103. Alternatively or additionally, the distal portion 110 of the elongate arm 106 may be positioned between the legs 103.

The aiming device 98 further includes an adjustment assembly 107 that is configured to adjust at least one of a directional (e.g., lateral) position and an angular orientation, collectively referred to herein as a position, of the elongate arm 106 relative to the base 100. The adjustment assembly 107 includes a biasing member 109 that is connected to and between the base 100 and the elongate arm 106. The biasing member 109 is configured as a spring 104, such as a plate spring or any alternative suitably constructed spring. The spring thus flexibly couples the proximal portion 108 of the elongate arm 106 to the base 100, for instance to the spacer 101, at a location between the opposed legs 103. The spring 104 can be configured as a leaf spring, as shown in Fig. 1A, a coil spring, or any suitable alternative spring or alternatively constructed biasing member as desired. The biasing member 109 is configured to allow motion of the elongate arm 106 in a particular direction. The biasing member 109 is disposed between the opposed legs 103, so as to flexibly connect the elongate arm 106 to the base 100. In accordance with the illustrated embodiment, the base 100 defines a slot 111 that extends transversely into or through the spacer 101. The slot 111 is configured to receive the biasing member 109 such that the biasing member 109 is secured in the slot 111. The biasing member 109 can define a plate 115 that can be supported by the base 100 in an orientation that is substantially planar in the longitudinal and transverse directions. Thus, the biasing member 109 is supported by the base 100 so as to be laterally flexible toward and away from each of the legs 103 and less flexible or substantially rigid with respect to the transverse direction T. Likewise, the elongate arm 106 can define a slot 113 that extends transversely into or through the proximal portion 108. The slots 111 and 113 are configured to receive opposed ends of the biasing member 109. It should be appreciated that the biasing member 109 can be flexibly connected between the base 100 and the elongate arm 106 in any suitable alternative manner as desired.

The elongate arm 106 includes a plurality of apertures 112 that extend through the distal portion 110. The apertures 112 can be configured to receive guiding sleeves, screwdrivers or other aids to place complementary screws, bolts, pins, pegs, or other locking members. The locking members in turn engage with apertures (which may be termed locking holes) disposed in an intramedullary nail that has been installed in a bone, as discussed and described elsewhere herein in further detail.

The apertures 112 of the elongate arm 106 may be of a diameter suitable for user with the appropriate locking screw. Such diameters can be in the range of from 1 mm to 20 mm, or from 2 mm to 18 mm, or from 5 mm to 15 mm, or even about 10 mm. The apertures may be spaced apart by regular increments, e.g., by 10 mm, 15 mm, 20 mm, or 25 mm. The spacing of the apertures 112 of the elongate arm 106 may depend, for instance, on the spacing between the apertures on the intramedullary nail being fixed, such that at least one such as a plurality of the apertures 112 can be selectively aligned with a corresponding at least one such as a plurality of apertures of the intramedullary nail.

The adjustment assembly 107 further includes at least one actuator such as a pair of actuators configured to bias the proximal portion 108 of the elongate arm 106 toward one of the opposed legs 103 and away from the other of the opposed legs 103. For instance, the actuators can include a respective one of a pair of knobs 116 and 120 and corresponding opposed movable members 114 and 118 that are attached to the knobs 116 and 120 and are configured to engage the elongate arm 106. For instance, the movable members 114 and 118 may be screws, bolts, and the like. The opposed movable members 114 and 118 can be supported by one of the legs 103 and laterally aligned with each other, such that a lateral axis extends through both movable members 114 and 118. It should be appreciated in accordance with an alternative embodiment that the movable members 114 and 118 can be laterally offset. The movable members 114 and 118 are configured to translate laterally so as to bear against the elongate arm 106, such as the proximal portion 108, and bias the elongate arm 106 toward the opposed leg 103. Thus, the movable members 114 can define pusher members configured to bias the elongate arm 106, such as the proximal portion 108 of the elongate arm 106, away from the respective leg 103 and at least one of laterally offset and angularly offset with respect to the central axis 140. Alternatively, the movable members 114 can be attached to the proximal portion so as to provide pull members that can pull the proximal portion 108 as they move laterally.

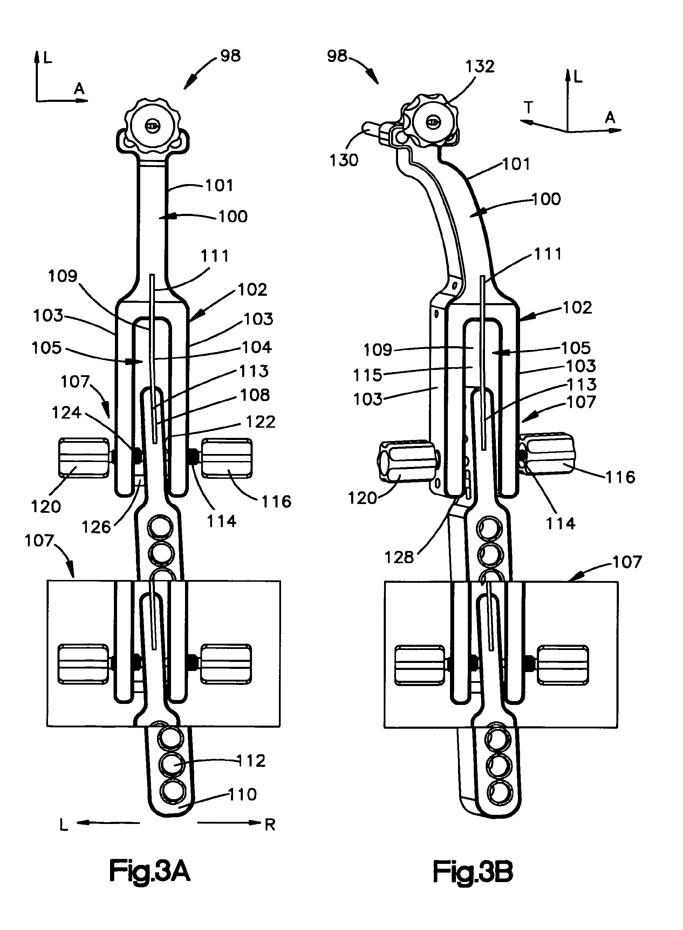

In accordance with the illustrated embodiment, the movable members 114 and 118 are threadedly supported by the legs 103 and rotatably coupled to the respective knobs 116 and 120, such that rotation of the knobs 116 and 120 causes the movable members 114 and 118 to correspondingly rotate. Accordingly, rotation of the respective knobs 116 and 120 in a first direction causes the movable members 114 and 118 to extend deeper into the gap 105, while rotation of the respective knobs 116 and 120 in an opposite second direction causes the movable members 114 and 118 to retract from the gap 105. Thus, each of the knobs can be selectively rotated (e.g, by turning) to adjust the position of the opposed movable members 114 and 118, which in turn adjusts the position of the elongate arm 106 and the deflection of the biasing member 109. For instance, the biasing member 109 can apply a force against the elongate arm 106 as it travels in response to the biasing force applied against the elongate arm 106 by the movable members 114 and 118, thereby retaining the arm, and in particular the proximal portion 108, against the movable members 114 and 118. The opposed movable members 114 and 118 may include an end 124 (see Fig. 3A), which can contact and apply a biasing force to the elongate arm 106. The opposed members 114 and 118 may alternatively or additionally be positioned to adjustably contact and apply a biasing force to the biasing member 109, such as the plate 115. Accordingly, the opposed movable members 114 and 118 may deflect the biasing member 109 directly, which in turn adjusts the position of the elongate arm 106 against the force of the spring 104. In both embodiments, it should be appreciated that the members 114 and 118 apply a biasing force to the elongate arm 106, for instance directly to the elongate arm 106 or indirectly via the biasing member 109.

The opposed movable members 114 and 118 can be adjusted along the lateral direction A so as to secure the elongate arm 106 in a desired position, for instance by moving at least one or both of the members 114 and 118 so as to achieve a desired deflection of the biasing member 109 or the elongate arm 106. For example, Figs. 3A-B illustrate that the knob 120 of the left-hand opposed member 118 has been turned so as to extend the opposed member 118 and thereby deflect the elongate arm 106 to the right. The right-hand opposed member 114 has been extended so as to engage and lock the spring 104 into position. Thus, one of the opposed members 114 and 118, such as the left-hand opposed member 118 as illustrated, can be adjusted so as to deflect the spring 104 and to place the elongate arm 106 into the desired position. Once the elongate arm 106 has been positioned, the other member 114, which can be referred to as a locking screw, may be tightened as shown in Fig. 3B so as to engage the elongate arm 106 and to lock the elongate am 106 into place.

The position of the elongate arm 106 may be accomplished in a number of ways. In one embodiment, the user may retract both opposed members 114 and 118 and then extend one of the opposed members 114 and 118 into the gap 105 so as to operatively engage the elongate arm 106 so as to apply a biasing force to the elongate arm 106 until the elongate arm 106 reaches the desired position. The user may then bring the other of the opposed member 114 and 118 against the elongate arm 106 so as to releasably lock the elongate arm 106 in the desired position. The opposed members 114 and 118 may have end caps 122 and 124, respectively, that are configured to contact the elongate arm 106. As shown in the illustrative figure, the opposed members 114 and 118 may be screws, and can thus be referred to herein as set screws. One or both of the caps 122 and 124 may be hardened. Alternatively, one or both of the caps 122 and 124 may be deformable.

The adjustment assembly 107 may also include a guide member 126 that guides movement of the elongate arm 106. For instance, the guide member 126 can be secured to either or both of the opposed legs 103 and configured to restrict rotation of the elongate arm 106 about the major axis of the elongate arm. The guide member 126 can be configured as a rod, a peg, or any suitable alternative shape as desired. The guide member 126 extends through a longitudinally elongate aperture 128 defined by in the elongate arm 106. That is, the elongate aperture 128 is elongate along a direction substantially parallel to the major axis of the elongate arm 106. As illustrated in Figs. 3A-B, guide member 126 engages the aperture 128 so as to allow the elongate arm 106 to deflect laterally (e.g., right or left in the illustrated orientation) and restrains the elongate arm 106 from deflecting into or outward from the plane of the figure. For instance, the guide member 126 permits deflection of the elongate arm and spring 104 in the lateral direction A, but limits or prevents deflection in the transverse and longitudinal directions T and L.

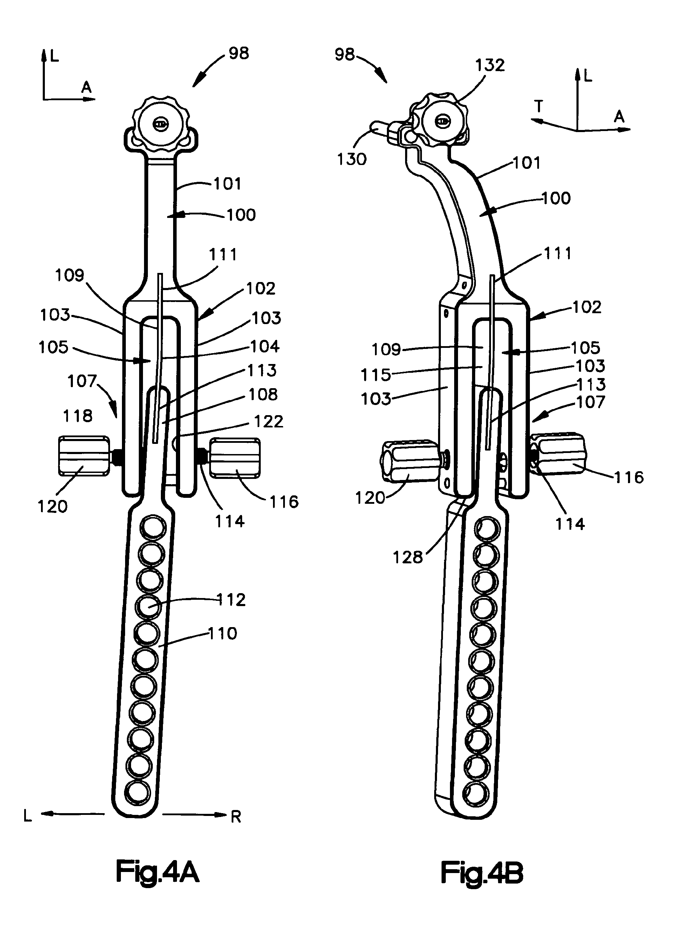

Referring to Figs. 4A-B, the aiming device 98 can be configured such that the elongate arm 106 and spring 104 are deflected to the left. To achieve this deflection, the right-hand opposed member 114 may be tightened (not shown) so as to deflect the elongate arm and spring 104 to the left. This deflection may also entail retracting the left-hand opposed member 118 so as to permit the desired amount of deflection of the spring 104. Once the spring 104 and elongate arm 106 are deflected, the left-hand opposed member 118 may be tightened so as to lock the arm (and spring) into place.

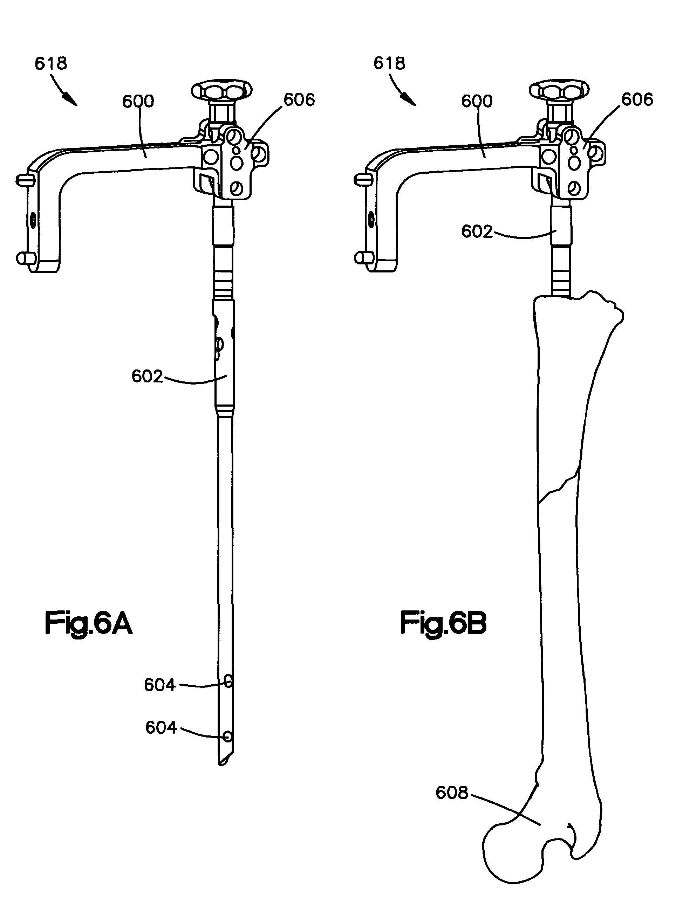

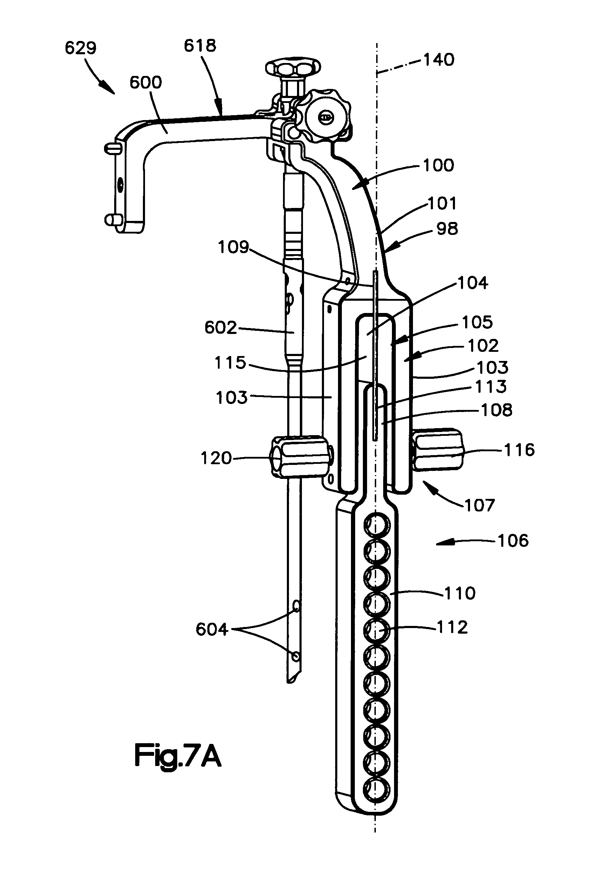

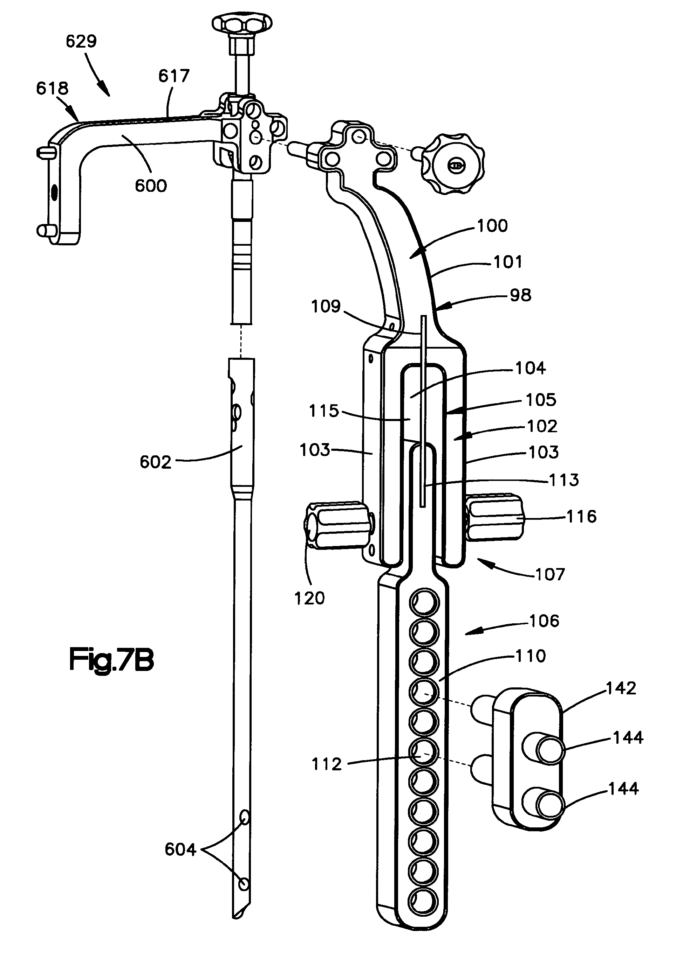

Referring now to Figs. 6A-7B, an implantation assembly 618 can include a support frame 600 and an intramedullary nail 602 that can be supported by the support frame 600, implanted into a long bone 608 such as a humerus, a femur, or the like, and subsequently detached from the support frame 600. The intramedullary nail 602 may include apertures 604 that receive screws, rods, pegs, or other fixation members to anchor the nail in the bone. The nail 602 may be attached to the support frame 600 in such a manner that the nail 602 extends substantially parallel to the central axis 140 of the elongate arm 106, as illustrated in Fig. 7 herein. The apertures 112 of the elongate arm 106 may be placed into alignment with one or more apertures 604 of the nail 602 when the nail 602 has been attached to the support frame 600 and implanted into the bone 608, such that bone anchors (e.g., a screw) driven through select ones of the apertures 112 of the elongate arm 106 can further be driven into or through corresponding select ones of the apertures 604 of the nail 602. It should be appreciated that an aiming assembly 629 can include the implantation assembly 618 and the aiming device 98 that can be coupled to the implantation assembly 618, for instance to the support frame 600. Accordingly, screws, nails, pegs, and the like may be inserted through the apertures 112 of the elongate arm 106 and mate with the intramedullary nail 602, for instance at the apertures 604. The apertures 604 of the nail may be at least partially or fully unthreaded, or at least partially or fully threaded so as to engage a complementary thread on a fixation member (e.g., a screw, peg) that is inserted through an aperture 112 of the elongate arm 106. The aperture 604 may also be splined so as to engage a complementary structure on a fixation member (e.g., a screw, peg) inserted through an aperture 112 of the elongate arm 106.

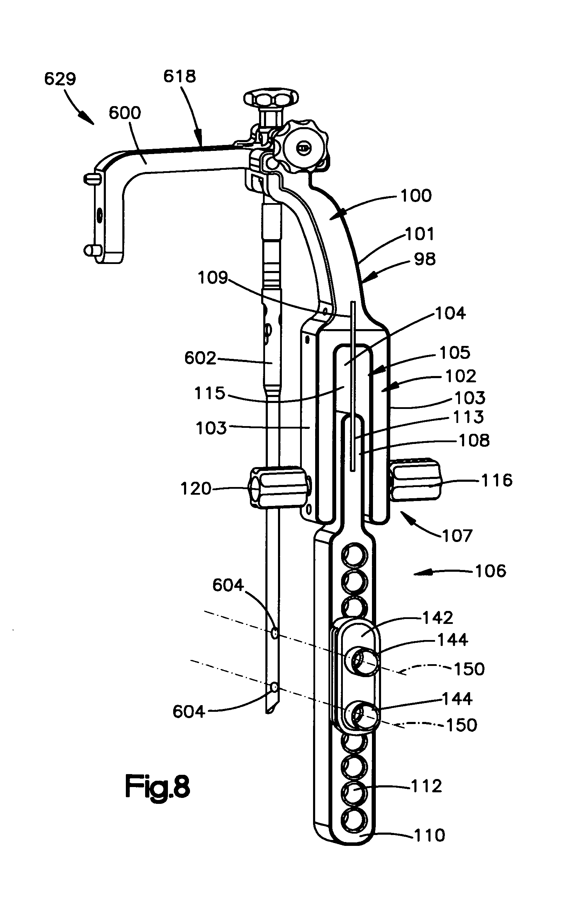

Referring also to Fig. 8, the aiming device 98 can also include a mask 142 that is configured to be coupled to the elongate arm 106, for instance at one or more of the apertures 112 of the elongate arm 106 so as to identify one or more select ones of the apertures 112 that are intended to receive a fastener so as to couple the elongate arm 106 to the nail 602. The mask 142 may be made of a material that differs (e.g., in color, in material) from the material of the elongate arm 106. This in turn assists the user in identifying which particular apertures 112 in the elongate arm 106 should be used to receive nails, screws, or other fixation members that anchor a nail into a patient. For example, the mask 142 may be made from a blue or red material, which in turn directs the user's attention to the mask apertures 144 that should be used for a given procedure.

The mask 142 may also include a radio-opaque material, which material allows the user to locate the mask on a radiographic image. The radio-opaque material of the mask 142 may be disposed about the edge or periphery of the mask 142 or at another predetermined location of the mask 142 to allow the user to locate the mask 142 on a radiographic image. The radio-opaque material may also be disposed around or near the apertures of the mask so as to facilitate the user's alignment of the apertures 144 relative to the apertures or fixation holes of the intramedullary nail.

The mask 142 may also include one or more projections that allow the user to seat the mask onto the elongate arm. Such projections may engage the elongate arm 106 and can further enter into apertures 112. The mask 142 may include one or more apertures 144 that are aligned with select ones of the apertures 112 of the elongate arm so as to identify those select apertures 112 that are aligned with complementary apertures 601 and 604 of the intramedullary nail 602. Thus, a radiographic image can identify the mask 142 and the apertures 112 that are operatively aligned with the complementary apertures 601 and 604 of the intramedullary nail 602. As shown by the figure, paths 150 illustrate that the mask apertures 144 are in register with apertures 112 of the elongate arm 106, which paths are followed by fixation members that are inserted through the elongate arm 106 of the aiming assembly into the nail 602.

While Fig. 8 illustrates a mask 142 having two apertures 144, the mask may have one, two, three, or more apertures 144 as desired. The aiming device 98 can include two or more masks 142 (e.g., at least one mask) that can be attached to the elongate arm 106, so as to draw the user's attention to particular apertures to be used in a surgical fixation procedure. For example, masks may be present to identify apertures at the most-distal portion of the elongate arm 106 and also to identify apertures at a less distal portion of the elongate arm. The mask 142 may engage with the elongate arm 106 with magnets. Alternatively, the mask 142 and elongate arm may have complementary peg-and-hole structures, tab-and-slot structures, or any suitable alternative fastening mechanism configured to attach (removably or permanently) the mask 142 to the elongate arm 106. The mask 142 may also movable with respect to the elongate arm 106. For instance, the mask 142 can translate along rails, ridges, or other structures (e.g., trenches, grooves) along elongate arm 106 along a direction substantially parallel to the central axis 140, thereby placing the at least one aperture 144 of the mask 142 in selective alignment with a corresponding at least one of the plurality of apertures 112 of the elongate arm 106.

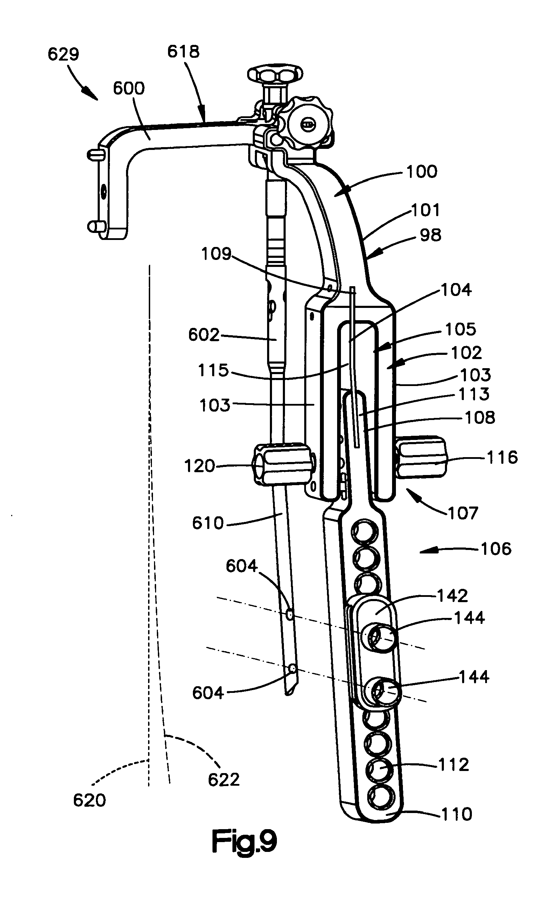

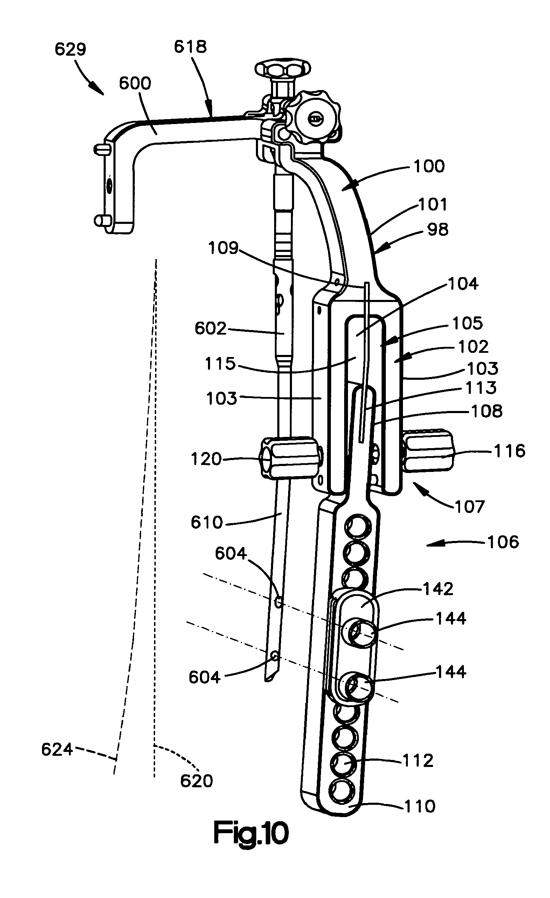

Referring now to Figs. 9-10, the nail 602 is shown laterally deflected (to the right as shown by deflection line 622 in Fig. 9, or to the left as shown by deflection line 624 in Fig. 10), for instance due to a curvature of the bone into which the nail 602 has been inserted that correspondingly deflects the nail 602 when the nail 602 is driven into the bone. Thus, the elongate arm 106 can likewise be laterally deflected to the right or left relative to the base 100 by adjustment of the opposed members 114 and 118 in the manner described above so that the mask apertures 144 and the arm apertures 112 are aligned with select ones of the apertures 604 of the nail 602. The angle of deflection of the nail 602 is defined between a line 620 that represents the direction of a non-deflected nail, which extend parallel to the central axis 140 of the base 100 of the aiming device 98) and the deflection line 622 or 624 that represents a central axis of the deflected nail 610. Thus, the actuators are configured to bias the proximal portion 108 of the elongate arm 106 toward one of the opposed legs 103 and away from the other of the opposed legs 103 so as to angularly adjust the major axis of the elongate arm 106 with respect to the central axis 140 of the base 100.

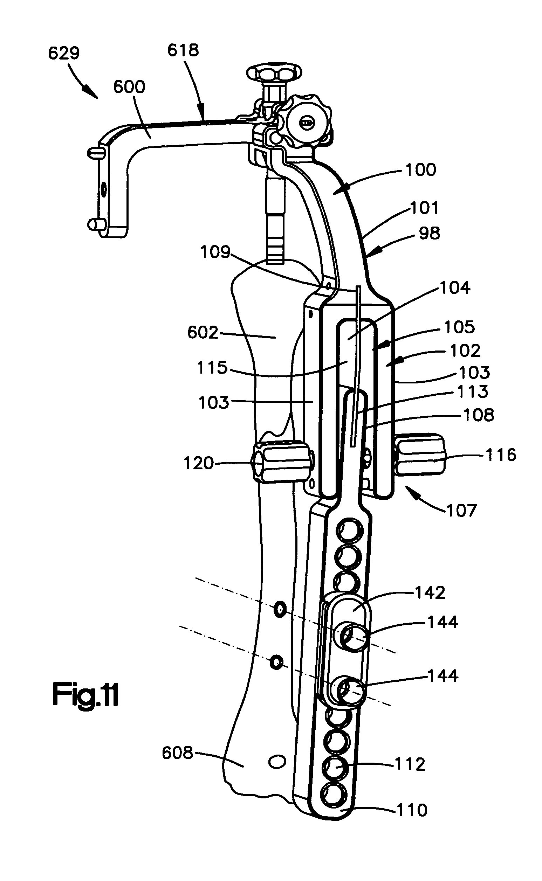

Referring to Fig. 11, the nail 602 is shown inserted into the long bone 608 in a deflected state as described above with respect to Fig. 10. Thus, the elongate arm 106 of the aiming device 98 can be correspondingly deflected such that the apertures 144 of the mask 142 and the apertures 112 of the elongate arm 106 are aligned with the apertures 604 of the nail 602, as illustrated by alignment paths 150 that show the trajectory of a fixation member (e.g., a screw, peg) that is inserted through apertures 112 of the elongate arm and apertures 144 of the mask 142 and into or through the apertures 604 of the nail 602 to anchor the nail 602 to the elongate arm 106.

During operation, the user may first insert a fixation nail (e.g., an intramedullary nail) into the target bone. This may be accomplished by a hammering, a driving, or by other methods known in the art. The nail may be inserted through support frame 600 (shown in Figs. 6A and 6B). The degree to which the nail is inserted may be preset by the user so that apertures or fixation holes 604 in the intramedullary nail will be in register or nearly in register with apertures on the elongate arm 106 of the aiming assembly.

The support frame 600 may be locked to the base 100 of the disclosed aiming assembly or to some other portion that is connected to the base. This connection may be made by way of a screw, peg, or other connector. The actuators are configured to adjust the deflection of the elongate arm may be one-piece screw-knob assemblies. The intramedullary nail 602 may be connected to a handle 617 by way of a screw, such as the connection screw shown in the figure. The nail is hammered or otherwise inserted into the medullary canal of the bone.

The base 100 may be curved, as shown in Fig. 14, to provide spatial separation between the aiming assembly and the installed nail. The base may be mated to the insertion handle 617 by way of screws, bolts, and the like, as shown in Fig. 14.

In the exemplary embodiment of Fig. 14, the mask includes extensions that insert into apertures on the elongate arm. The extensions, when inserted into the elongate, stabilize the mask and fix it into position. The collars/markers on the mask may be made of a radio-opaque material and can direct the user's attention to the specific apertures through which the fixation screws will be inserted into the patient and into the fixation holes of the nail.

Once the user has installed the nail into the bone, the nail may deflect as a result of following the natural curvature of the bone. The user may then modulate the opposed members so as to adjust the deflection of the elongate arm to compensate for the deflection of the nail. Thus, the deflection of the elongate arm can be adjusted so as to place the apertures of the elongate arm into register with apertures of the installed nail. The user may also translate the elongate arm in the direction of the major axis of the nail so as to more precisely align the apertures.

The base 100 can be slidably mounted to the insertion handle 617. For instance, the base 100 can be attached to the insertion handle 617 by a screw, a clip, or other attachment member. For instance, the base 100 can be secured to the handle 617 by screws. The base 100 is then capable of being translated relative to the nail 602 along a direction parallel to the major axis of the nail 602.

Once the user placed apertures of the elongate arm 106 in register with corresponding apertures of the intramedullary nail 602, the user may perform a stab or other suitable incision through or in register with the appropriate aperture. The user may then insert a drill sleeve/trocar through the aperture and advance to the patient's bone. The user may then drill a hole in the bone for insertion therethrough of a locking screw (for instance after confirming that the drill sleeve is oriented so as to point directly at the nail's aperture or locking hole). The user may then repeat this process to form holes for additional locking screws as needed. Once the holes are formed in the bone, the user may then insert appropriate locking screws according to standard methods and engage the screws with the apertures of the nail.

Thus, in accordance with one embodiment, a method is provided for aligning at least one aperture of an aiming device with an aperture of an intramedullary nail. The aiming device can include a base and at an elongate arm that defines the aperture of the aiming device as described above. The method can include the step of inserting the intramedullary nail into a medullary canal, actuating a movable member that is supported by the base of the aiming device, and, in response to the actuating step, biasing the elongate arm from a first position to a second deflected position against a force of a spring that is coupled between the base and the elongate arm, such that the at least one aperture of the aiming device is aligned with the aperture of the intramedullary nail when the elongate arm is in the deflected position. The base can include a spacer and a pair of opposed legs that extend from the spacer and define a gap therebetween, and the actuating step can further include the step of rotating a knob so as to thread the movable member through one of the opposed legs toward the other of the opposed legs. The method can further include the step of attaching a radio-opaque mask to the elongate arm, the mask defining an aperture that is aligned with the at least one aperture of the aiming device after the attaching step.

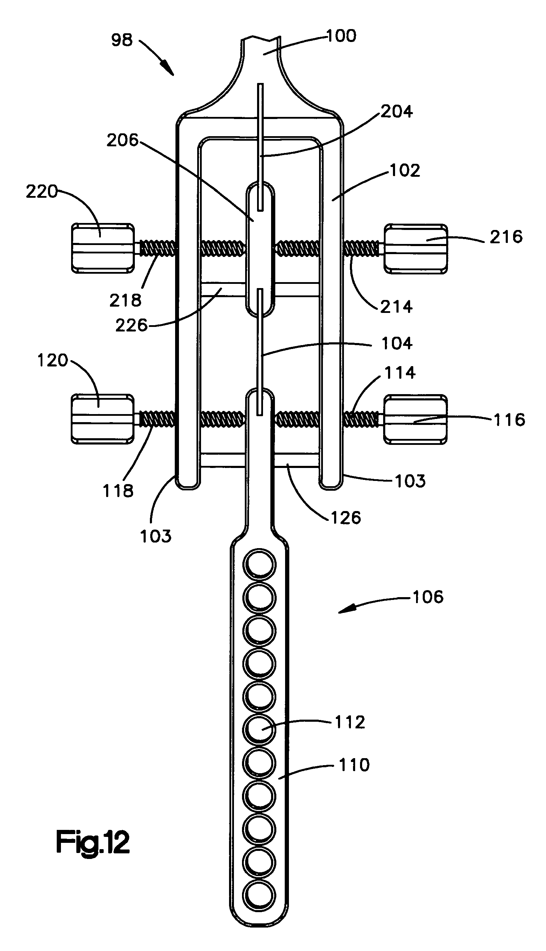

Referring now to Fig. 12, the aiming device 98 constructed in accordance with another embodiment can include a base 100 that includes a spacer and a forked projection 102 that extends distally from the spacer 101 and can thus define the distal end of the base 100. The forked projection 102 includes a pair of laterally spaced legs 103 in the manner described above. The aiming device 98 includes a first biasing member such as a first spring 104 that flexibly couples the proximal portion 108 of the elongate arm 106 to the base 100 at a location between the opposed legs 103. In accordance with the illustrated embodiment, the first spring 104 is attached to the elongate arm 106, for instance at the proximal portion 108. The aiming device 98 can further include an intermediate link 206 that is flexibly coupled between the base 100 and the first spring 104 (and thus between the base 100 and the proximal portion 108 of the elongate arm 106). For instance, the aiming device 98 can further include a second biasing member such as a second spring 204 that can be configured as a plate spring or any alternative suitably constructed spring. The second spring 204 can be connected between the intermediate link 206 and the base 100. For instance, the second spring 204 can be attached to the base 100 at one end, and attached to the intermediate link 206 at an opposite end. Accordingly, the intermediate link 206 is flexibly connected between the base 100 and the elongate arm 106. For instance, the first spring 104 can flexibly connect the intermediate link 206 to the elongate arm 106, and the second spring 204 can flexibly connect the intermediate link 206 to the base 100. It can further be said that the intermediate link 206 is flexibly supported between the base 100 and the elongate arm 106. Thus, the intermediate link 206 can flex, for instance along the lateral direction A, with respect to the base 100, for instance toward one of the opposed legs 103 and away from the other of the opposed legs 103.

The aiming device 98 can further include a first one first guide member 126 that is attached to at least one of the legs 103 and extends at least into or through an aperture of the elongate arm 106 so as to prevent or limit rotation of the elongate arm in the manner described above. The aiming device 98 can further include at least one second guide member 226 that is attached to at least one of the legs 103 can extends at least into or through an aperture of the intermediate link 206. The second guide member 226 is configured to constrain the motion of the intermediate link 206, as described above, so as to prevent the intermediate link 206 from rotating about its major axis.

The aiming device 98 can further include at least one first actuator, such as a pair of first actuators that can include respective knobs 116 and 120 and corresponding opposed movable members 114 and 118 that are attached to the knobs 116 and 120 and are configured to engage the elongate arm 106 so as to secure the elongate arm 106 in a desired position as described above. The aiming device 98 can further include at least one second actuator, such as a pair of second actuators that can include respective knobs 216 and 220 (e.g., adjustable screws) that are supported by the opposed legs 103, respectively. The at least one second actuator further includes opposed movable members 214 and 218 that are attached to the respective knobs 216 and 220 and extend through the respective opposed legs 103. The movable members 214 and 218 are configured to bear against and engage the intermediate link 206 so as to secure the intermediate link 206 in a desired position as described above with respect to the at least one first actuator, and to deflect the intermediate link toward one of the opposed legs 103 and away from the other of the opposed legs 103 in the manner described above with respect to the at least one first actuator. Thus, the opposed movable members 214 and 218 may be adjusted along the lateral direction A by rotating knobs 216 and 220, which causes one of the opposed movable members 214 and 218 to bias the intermediate link 206 toward one of the opposed legs 103 and away from the other of the opposed legs 103 against the spring force of the first and second springs 104 and 204. It should be appreciated that the at least one first actuator and the at least one second actuator are configured to deflect the respective elongate arm 106 and the intermediate link 206 independently of each other.

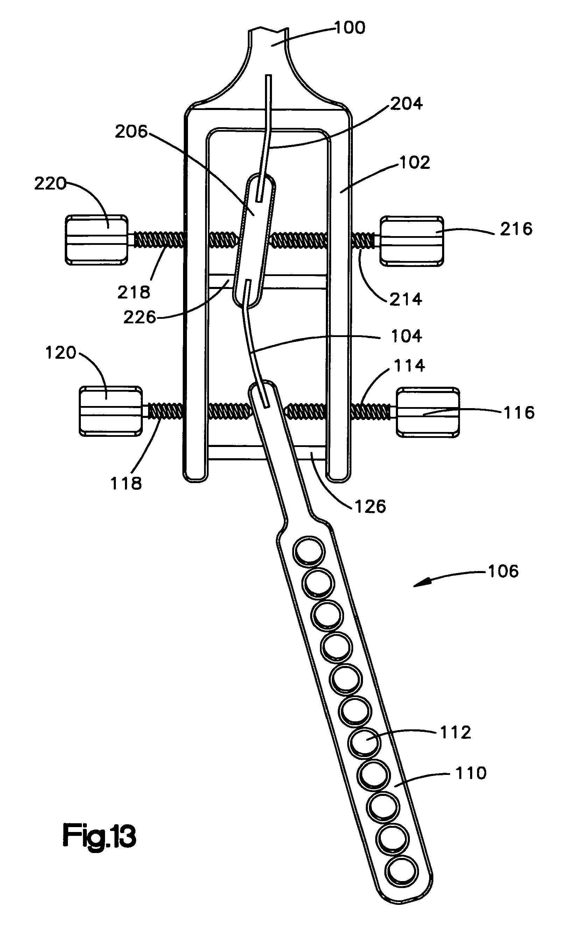

As illustrated in Fig. 13, at least one of or both of the intermediate link 206 and the elongate arm 106 can be deflected, for instance along the lateral direction A (thus toward one of the opposed legs 103 and away from the other of the opposed legs 103). The intermediate link 206 and the elongate arm 106 can be deflected by the same amount, by different amounts, in the same direction, or in different directions. In accordance with the illustrated embodiment in Fig. 13, the first set of opposed members 214 and 218 have been adjusted so as to deflect the intermediate link 206 in one lateral direction (to the left as illustrated). The second set of opposed members 114 and 118 have been adjusted so as to deflect the elongate arm 106 in an opposite lateral direction (to the right as illustrated). It should thus be appreciated that the intermediate link 206, the elongate arm 106, and the multiple sets of opposed members define a pair of laterally deflectable joints that allow the apertures of the elongate arm 106 to be positioned as desired, for instance when aligned with corresponding apertures of an intramedullary nail that has been inserted into a curved bone. In this regard, it should be appreciated that the aiming device 98 can define at least one laterally deflectable joint that can be actuated so as to align the apertures 112 with respective apertures of an intramedullary nail.

The aiming device 98 and components thereof can be made from any suitable material. For instance, at least one or both of the base 100, and forked projection 102, and the elongate arm 106 may be metallic (e.g., titanium, stainless steel), made from a plastic (e.g., polymethylmethacrylate, polycarbonate, PEEK).

The foregoing description is provided for the purpose of explanation and is not to be construed as limiting the invention. While various embodiments have been described with reference to preferred embodiments or preferred methods, it is understood that the words which have been used herein are words of description and illustration, rather than words of limitation. Furthermore, although the embodiments have been described herein with reference to particular structure, methods, and embodiments, the invention is not intended to be limited to the particulars disclosed herein. Furthermore, it should be appreciated that the structure and methods as described with one embodiment can be included in all other embodiments unless otherwise indicated. Those skilled in the relevant art, having the benefit of the teachings of this specification, may effect numerous modifications to the invention as described herein, and changes may be made without departing from the scope of the invention as defined by the appended claims.

## Claims

1. An aiming assembly (98), comprising:
a base (100) that is elongate along a central axis (140) that extends along a longitudinal direction (L), the base (100) having a spacer member (101) and a forked projection (102) that extends from the spacer member (101), the forked projection including a pair of opposed legs (103) that are spaced from each other along a lateral direction (A) that is substantially perpendicular to the longitudinal direction (L) so as to define a gap (105) between the opposed legs (103);
an elongate arm (106) defining a proximal portion (108) and a distal portion (110) that is spaced from the proximal portion (108) along a major axis of the elongate arm (106), wherein the proximal portion (108) is at least partially disposed in the gap (105), and the elongate arm (106) defines a plurality of apertures (112) that extend through the distal portion (110);
a spring (104) that flexibly couples the proximal portion (108) of the elongate arm (106) to the base (100) at a location between the opposed legs (103); and
at least one actuator (114, 118) that is configured to bias the proximal portion (108) of the elongate arm (106) toward one of the opposed legs (103) and away from the other of the opposed legs (103) against a force of the spring (104).

2. The aiming assembly as recited in claim 1, wherein the central axis (140) extends between the opposed legs (103), and the at least one actuator (114, 118) is configured to angularly adjust the major axis of the elongate arm (106) with respect to the central axis (140) of the base (100).

3. The aiming assembly as recited in claim 1 or 2, wherein the spring (104) is attached to the elongate arm (106).

4. The aiming assembly as recited in claim 3, wherein the spring (104) is further attached to the base (100).

5. The aiming assembly as recited in any one of claims 1 to 4, wherein the at least one actuator (114, 118) comprises a screw extending through one of the opposed legs (103).

6. The aiming assembly as recited in any one of claims 1 to 5, further comprising a guide member (126) attached to the forked projection (102) and engaging the elongate arm (106) so as to restrict rotation of the elongate arm (106) about the major axis of the elongate arm (106), wherein preferably the guide member (126) extends at least into an aperture (128) of the elongate arm (106).

7. The aiming assembly as recited in any one of claims 1 to 6, wherein the base (100) engages with a handle (600) that is configured to accept an intramedullary nail (602) inserted into the handle (600) such that the intramedullary nail (602) is essentially parallel to the major axis of the elongate arm (106).

8. The aiming assembly as recited in claim 7, wherein the intramedullary nail (602) comprises one or more apertures (604).

9. The aiming assembly as recited in claim 8, wherein one or more apertures (112) of the elongate arm (106) are capable of being placed into register with one or more apertures (604) of the intramedullary nail (602) when the intramedullary nail (602) is inserted into the handle (600).

10. The aiming assembly as recited in claim 9, further comprising an aperture mask (142) configured to engage at least one of the apertures (112) of the elongate arm (106), the aperture mask (142) configured to be placed in alignment with a corresponding aperture (604) of an intramedullary nail (602).

11. The aiming assembly as recited in claim 10, wherein the mask (142) is radio-opaque.

12. The aiming assembly as recited in any one of claims 1 to 11, further comprising an intermediate link (206) disposed in the gap (105), wherein the spring is a first spring (104) that is coupled between the intermediate link (206) and the elongate arm (106).

13. The aiming assembly as recited in claim 12, further comprising a second spring (204) connected between the base (100) and the intermediate link (206).

14. The aiming assembly as recited in claim 13, wherein the at least one actuator is at least one first actuator (114, 118), the aiming assembly (98) further comprising at least one second actuator (214, 218) that is configured to bias the intermediate portion (206) toward one of the opposed legs (103) and away from the other of the opposed legs (103).

15. The aiming assembly as recited in claim 14, wherein the first and second actuators (114, 118, 214, 218) comprise movable members threadedly supported by respective ones of the first and second legs (103).

## Patentansprüche

1. Zielanordnung (98), umfassend:
eine Basis (100), die entlang einer Mittelachse (140) länglich ist, die sich entlang einer Längsrichtung (L) erstreckt, wobei die Basis (100) einen Abstandshalter (101) und eine gabelförmige Verlängerung (102) aufweist, die sich von dem Abstandshalter (101) erstreckt, wobei die gabelförmige Verlängerung ein Paar von gegenüberliegenden Beinen (103) enthält, die voneinander entlang einer lateralen Richtung (A) beabstandet sind, die im Wesentlichen senkrecht zur Längsrichtung (L) ist, um eine Lücke (105) zwischen den gegenüberliegenden Beinen (103) zu definieren;
einen länglichen Arm (106), der einen proximalen Teil (108) und einen distalen Teil (110), der von dem proximalen Teil (108) entlang einer Hauptachse des länglichen Arms (106) beabstandet ist, definiert, wobei der proximale Teil (108) zumindest teilweise in der Lücke (105) angeordnet ist, und der längliche Arm (106) eine Mehrzahl von Öffnungen (112) definiert, die sich durch den distalen Teil (110) hindurch erstrecken;
eine Feder (104), die den proximalen Teil (108) des länglichen Arms (106) mit der Basis (100) an einer Stelle zwischen den gegenüberliegenden Beinen (103) flexibel koppelt; und
zumindest eine Betätigungseinrichtung (114, 118), die eingerichtet ist, den proximalen Teil (108) des länglichen Arms (106) in Richtung eines der gegenüberliegenden Beine (103) und weg von dem anderen der gegenüberliegenden Beine (103) gegen eine Kraft der Feder (104) zu drängen.

2. Zielanordnung nach Anspruch 1, wobei sich die Mittelachse (140) zwischen den gegenüberliegenden Beinen (103) erstreckt und die zumindest eine Betätigungseinrichtung (114, 118) eingerichtet ist, die Hauptachse des länglichen Arms (106) bezüglich der Mittelachse (140) der Basis (100) winklig einzustellen.

3. Zielanordnung nach Anspruch 1 oder 2, wobei die Feder (104) an dem länglichen Arm (106) angebracht ist.

4. Zielanordnung nach Anspruch 3, wobei die Feder (104) des Weiteren an der Basis (100) angebracht ist.

5. Zielanordnung nach einem der Ansprüche 1 bis 4, wobei die zumindest eine Betätigungseinrichtung (114, 118) eine Schraube umfasst, die sich durch eines der gegenüberliegenden Beine (103) hindurch erstreckt.

6. Zielanordnung nach einem der Ansprüche 1 bis 5, des Weiteren umfassend ein Führungselement (126), das an der gabelförmigen Verlängerung (102) angebracht ist und mit dem länglichen Arm (106) zusammenwirkt, um eine Rotation des länglichen Arms (106) um die Hauptachse des länglichen Arms (106) zu beschränken, wobei sich das Führungselement (126) vorzugsweise zumindest in eine Öffnung (128) des länglichen Arms (106) erstreckt.

7. Zielanordnung nach einem der Ansprüche 1 bis 6, wobei die Basis (100) mit einem Griff (600) zusammenwirkt, der eingerichtet ist, einen Marknagel (602) aufzunehmen, der in den Griff (600) eingeführt wird, so dass der Marknagel (602) im Wesentlichen parallel zur Hauptachse des länglichen Arms (106) ist.

8. Zielanordnung nach Anspruch 7, wobei der Marknagel (602) eine oder mehrere Öffnungen (604) umfasst.

9. Zielanordnung nach Anspruch 8, wobei eine oder mehrere Öffnungen (112) des länglichen Arms (106) geeignet sind, registerhaltig mit einer oder mehreren Öffnungen (604) des Marknagels (602) platziert zu werden, wenn der Marknagel (602) in den Griff (600) eingeführt ist.

10. Zielanordnung nach Anspruch 9, des Weiteren umfassend eine Öffnungsmaske (142), die eingerichtet ist, mit zumindest einer der Öffnungen (112) des länglichen Arms (106) zusammenzuwirken, wobei die Öffnungsmaske (142) eingerichtet ist, zu einer korrespondierenden Öffnung (604) eines Marknagels (602) ausgerichtet platziert zu werden.

11. Zielanordnung nach Anspruch 10, wobei die Maske (142) röntgendicht ist.

12. Zielanordnung nach einem der Ansprüche 1 bis 11, des Weiteren umfassend eine Zwischenverbindung (206), die in der Lücke (105) angeordnet ist, wobei die Feder eine erste Feder (104) ist, die zwischen der Zwischenverbindung (206) und dem länglichen Arm (106) gekoppelt ist.

13. Zielanordnung nach Anspruch 12, des Weiteren umfassend eine zweite Feder (204), die zwischen der Basis (100) und der Zwischenverbindung (206) verbunden ist.

14. Zielanordnung nach Anspruch 13, wobei die zumindest eine Betätigungseinrichtung zumindest eine erste Betätigungseinrichtung (114, 118) ist, wobei die Zielanordnung (98) des Weiteren zumindest eine zweite Betätigungseinrichtung (214, 218) umfasst, die eingerichtet ist, den Zwischenteil (206) in Richtung eines der gegenüberliegenden Beine (103) und weg von dem anderen der gegenüberliegenden Beine (103) zu drängen.

15. Zielanordnung nach Anspruch 14, wobei die erste und zweite Betätigungseinrichtung (114, 118, 214, 218) bewegliche Elemente umfassen, die gewindeartig von entsprechenden des ersten und zweiten Beins (103) gehalten werden.

## Revendications

1. Ensemble de visée (98), comprenant :
une base (100), allongée le long d'un axe central (140) qui s'étend le long d'une direction longitudinale (L), la base (100) présentant un élément espaceur (101) et une saillie à fourche (102) qui s'étend à partir de l'élément espaceur (101), la saillie à fourche incluant une paire de jambes opposées (103) qui sont espacées l'une de l'autre le long d'une direction latérale (A) qui est sensiblement perpendiculaire à la direction longitudinale (L), de manière à définir un espace (105) entre les jambes opposées (103) ;
un bras allongé (106) définissant une partie proximale (108) et une partie distale (110) qui est espacée de la partie proximale (108) le long d'un grand axe du bras allongé (106), dans lequel la partie proximale (108) est au moins en partie disposée dans l'espace (105), et le bras allongé (106) définit une pluralité d'ouvertures (112) qui s'étendent à travers la partie distale (110) ;
un ressort (104) qui couple de manière flexible la partie proximale (108) du bras allongé (106) à la base (100) en un endroit entre les jambes opposées (103), et
au moins un actionneur (114, 118) configuré de manière à solliciter la partie proximale (108) du bras allongé (106) vers l'une des jambes opposées (103) et à distance de l'autre des jambes opposées (103) à l'encontre d'une force du ressort (104).

2. Ensemble de visée selon la revendication 1, dans lequel l'axe central (140) s'étend entre les jambes opposées (103) et le au moins un actionneur (114, 118) est configuré pour régler de manière angulaire le grand axe du bras allongé (106) par rapport à l'axe central (140) de la base (100).

3. Ensemble de visée selon la revendication 1 ou 2, dans lequel le ressort (104) est fixé au bras allongé (106).

4. Ensemble de visée selon la revendication 3, dans lequel le ressort (104) est en outre fixé à la base (100).

5. Ensemble de visée selon l'une quelconque des revendications 1 à 4, dans lequel le au moins un actionneur (114, 118) comprend une vis s'étendant à travers une des jambes opposées (103).

6. Ensemble de visée selon l'une quelconque des revendications 1 à 5, comprenant en outre un élément de guidage (126) fixé à la saillie à fourche (102) et venant en prise avec le bras allongé (106) de manière à restreindre la rotation du bras allongé (106) autour du grand axe du bras allongé (106), dans lequel l'élément de guidage (126) s'étend de préférence au moins jusque dans une ouverture (128) du bras allongé (106).

7. Ensemble de visée selon l'une quelconque des revendications 1 à 6, dans lequel la base (100) vient en prise avec une poignée (600) configurée pour accepter un clou intramédullaire (602) introduit dans la poignée (600) de telle sorte que le clou intramédullaire (602) soit en grande partie parallèle au grand axe du bras allongé (106).

8. Ensemble de visée selon la revendication 7, dans lequel le clou intramédullaire (602) comprend une ou plusieurs ouvertures (604).

9. Ensemble de visée selon la revendication 8, dans lequel une ou plusieurs ouvertures (112) du bras allongé (106) sont en mesure d'être placées de manière à coïncider avec une ou plusieurs ouvertures (604) du clou intramédullaire (602) lorsque le clou intramédullaire (602) est introduit dans la poignée (600).

10. Ensemble de visée selon la revendication 9, comprenant en outre un masque perforé (142) configuré pour venir en prise avec au moins une des ouvertures (112) du bras allongé (106), le masque perforé (142) étant configuré pour être placé en alignement avec une ouverture correspondante (604) d'un clou intramédullaire (602).

11. Ensemble de visée selon la revendication 10, dans lequel le masque (142) est radio-opaque.

12. Ensemble de visée selon l'une quelconque des revendications 1 à 11, comprenant en outre un maillon intermédiaire (206) disposé dans l'espace (105), dans lequel le ressort est un premier ressort (104) qui est couplé entre le maillon intermédiaire (206) et le bras allongé (106).

13. Ensemble de visée selon la revendication 12, comprenant en outre un second ressort (204) relié entre la base (100) et le maillon intermédiaire (206).

14. Ensemble de visée selon la revendication 13, dans lequel le au moins un actionneur est au moins un premier actionneur (114, 118), l'ensemble de visée (98) comprenant en outre au moins un second actionneur (214, 218) configuré pour solliciter la partie intermédiaire (206) vers une des jambes opposées (103) et à distance de l'autre des jambes opposées (103).

15. Ensemble de visée selon la revendication 14, dans lequel les premier et second actionneurs (114, 118, 214, 218) comprennent des éléments mobiles supportés par filetage par des jambes respectives parmi les première et seconde jambes (103).
